# EUROPEAN PATENT APPLICATION

(11) **EP 2 120 050 A1**
(43) Date of publication of application: **18.11.2009**
(21) Application number: 08009042.6
(22) Date of filing: 15.05.2008
(51) Int. Cl.: G01N 33/68, C07K 14/435

(54) **Identification of ligand recognition domains**

(71) Applicant: Steinbeis-Transferzentrum Biopolymeranalytik/Proteomics und Proteinchemie, 78464 Konstanz (DE)
(72) Inventor: Przyblyski, Michael, Prof. Dr., 78465 Konstanz (DE); Moise, Adrian, Dipl.-Chem., 78464 Konstanz (DE); Gabius, Hans-Joachim, Prof. Dr., 83071 Stephanskirchen (DE)
(74) Representative: Müller-Boré & Partner Patentanwälte

(57) **Abstract**

The present invention relates to methods for the identification of ligand recognition domains (LRD) in ligand-binding proteins or the identification of ligand-containing molecules, to diagnostic assays and kits for carrying out said methods and to peptides comprising LRD sequences.

## Description

The present invention relates to methods for the identification of ligand recognition domains (LRD) in ligand-binding proteins or the identification of ligand-containing molecules, to diagnostic assays and kits for carrying out said methods and to peptides comprising LRD sequences.

The vast majority of proteins has the ability to specifically interact with one or more ligands. Such proteins include not only enzymes interacting with a substrate, but also, for example, transport proteins, receptors, and transcription factors. Two very important classes of ligands for proteins are carbohydrates and nucleic acids. Interactions of proteins with carbohydrates or nucleic acids play an important role in a multitude of physiological and cellular processes such as cellular recognition processes, intracellular regulation pathways, immunological reactions and the transcriptional or post-transcriptional regulation of gene expression. The identification and/or determination of the LRD of a protein binding such ligands is a prerequisite for the thorough mechanistic understanding of that protein's function and for the development of potential biochemical, analytical or biomedical applications involving that protein.

The complete structures of proteins with their bound ligand have been determined in a few cases both by X-ray crystallography and by nuclear magnetic resonance (NMR) spectroscopy, e.g. for oligo- and polysaccharide complexes bound to lectins. To accurately identify the position and the mode of binding of a ligand, a resolution of at least 2 to 2.5 Å is required. Such a resolution is often difficult or impossible to obtain, and in general crystallisation of protein-ligand complexes suitable for structure and interaction determinations is a tedious and time-demanding process. In some cases, a crystal structure can be obtained for a ligand-binding protein independent of the ligand, and its structure and potential binding site can be predicted using information from the three-dimensional structure of a homologous protein. For the determination of the structure of biomolecules and their interactions in solution, NMR is often a preferred technique. In NMR, the proton-proton distances can be obtained following assignment of the proton resonances through multidimensional techniques, such as the nuclear Overhauser effect (NOE). This information coupled with computational methods employing computer modelling allows the prediction of e.g. glycan conformations in solution. It is possible to extend this information to examine the interaction of e.g. a glycan with a protein in solution using transfer NOE (TRNOE).

However, this approach has a number of premises and limitations, and requires relatively large amounts of purified samples. Available computer-assisted modelling strategies and information from, e.g. glycan solution conformations and protein three-dimensional structures can be combined with NMR. Although these approaches can be highly informative, they are often limited by the requirement of relatively large amounts of highly purified material and by the problem that many of these interactions are performed under non-equilibrium conditions. Hence, a molecular chemical method capable of identifying the binding structures at high molecular specificity and sensitivity (low sample amounts), and with lower requirements in purity is needed.

Direct methods of chemical structure identification of biological ligand complexes such as protein-peptide interactions, receptor-ligand complexes and antigenic determinants (epitopes) in peptide/protein-antibody complexes using combinations of selective proteolytic and affinity mass spectrometric methods have been developed since several years, for example the isolation of antibody-bound peptides using immuno-affinity techniques followed by the precise identification of epitope peptides by mass spectrometry. Mass spectrometric methods can provide structural details on many levels for different classes of biomolecules. Proteins can now be analyzed by mass spectrometry to reveal elemental composition, complete or partial amino acid sequences, post-translational modifications, protein-protein interaction sites, and even provide insight into higher order structure. One key advantage of mass spectrometric analyses over other analytical techniques is its capability to study extremely small quantities of molecules with high sensitivity. The "soft" ionization/desorption techniques such as electrospray (ESI) and matrix assisted laser desorption/ionization (MALDI) are major tools for these analyses.

In summary, major problems and limitations of present methods for the elucidation of protein structures, with or without bound ligands, are *inter alia* due to
(i) high requirements regarding the purity of proteins for obtaining X-ray crystal structure determinations or NMR structure analyses;
(ii) large sample amounts needed in pure/homogeneous form, which may make difficult, limit or obviate the application of these methods to biological materials; and
(iii) NMR and X-ray crystal structure analyses of protein-ligand complexes are time-consuming and require elaborate and lengthy experimental protocols.

Thus, the technical problem underlying the present invention is to provide a novel method for the identification and/or determination of ligand recognition domains in ligand-binding proteins requiring a low amount of input material which may have a lower purity which can be carried out maintaining the native structure of the proteins with high accuracy.

The solution to the above technical problem is achieved by the embodiments characterized in the claims.

In particular, the present invention relates to a method for the identification and/or determination of at least one ligand recognition domain (LRD) of a ligand-binding proteinaceous compound, comprising the steps of:
(a) contacting said proteinaceous compound with a proteolytically stable ligand immobilized on a matrix under conditions suitable for binding said proteinaceous compound to said ligand;
(b) proteolytically degrading the proteinaceous compound bound to the immobilized ligand of step (a);
(c) releasing the ligand-bound LRD-containing fragment(s) of the proteinaceous compound obtained in step (b) from the ligand; and
(d) analyzing the fragment(s) obtained in step (c) using mass spectrometry (MS).

In another aspect, the present invention relates to a diagnostic assay for detecting a disorder associated with an increase or decrease of a ligand-binding proteinaceous compound and/or with an altered ligand recognition of a ligand-binding proteinaceous compound, comprising the steps of:
(a) contacting a sample containing a proteinaceous compound with a proteolytically stable ligand immobilized on a matrix under conditions suitable for binding said proteinaceous compound to said ligand;
(b) proteolytically degrading the proteinaceous compound bound to the immobilized ligand of step (a);
(c) releasing the ligand-bound ligand recognition domain (LRD)-containing fragment(s) of the proteinaceous compound, obtained in step (b) from the ligand; and
(d) analyzing the fragment(s) obtained in step (c) using mass spectrometry (MS).

In one embodiment of the present invention, the disorder associated with an increase or decrease of a ligand-binding proteinaceous compound and/or with an altered ligand recognition of a ligand-binding proteinaceous compound is associated with an increase or decrease in abundance or concentration of a ligand-binding proteinaceous compound.

Further, in another embodiment of the present invention, the LRD is defined as a partial amino acid sequence of the ligand-binding proteinaceous compound.

The term "ligand" as used herein relates to any compound having the ability to specifically interact with a certain proteinaceous compound resulting in a binding of the proteinaceous compound to the ligand. The ligand used herein is proteolytically stable, which means that it does not decompose in step (b) of the above method, or decomposes at a significantly slower rate, and is a more stable ligand than the binder under the assay conditions. In a preferred embodiment of the present invention, the ligand according to the present invention comprises a carbohydrate moiety. In a more preferred embodiment of the present invention, the carbohydrate moiety is selected from the group consisting of monomeric carbohydrates, dimeric carbohydrates, linear polymeric carbohydrates and branched polymeric carbohydrates. Examples of such a carbohydrate moiety are lactose; monosaccharides and their N-acetylated derivatives, like galactose, glucose, mannose, fucose, N-acetylglucosamine and N-acetyl-galactosamine; sialic acids, like N-acetylneuraminic acid and related carbohydrates; disaccharides and their N-acetylated derivatives, like saccharose, lactose, N-acetyl-lactosamine; blood group A trisaccharide, blood group B trisaccharide, blood group H trisaccharide; blood group A tetrasaccharide, blood group B tetrasaccharide; Man5 and other branched oligosaccharides.

In another preferred embodiment of the present invention, the ligand according to the present invention comprises a nucleic acid moiety. In a more preferred embodiment of the present invention, the nucleic acid moiety is selected from the group consisting of double-stranded (ds) DNA, single stranded (ss) DNA, ssRNA, dsRNA, mRNA, tRNA, siRNA, rRNA, DNA aptamers, RNA aptamers, or natural and synthetic G - A - T - C combinations of any sequence.

The ligand used in the method according to the present invention is not limited to any structural properties such as the length of a nucleic acid or the bulk of carbohydrate as long as the structural properties of the ligand do not have any negative impact on the binding of the ligand-binding proteinaceous compound.

The term "ligand recognition domain" as used herein relates to any part of the proteinaceous compound being in direct, full or partial physical contact to the ligand, for example a certain portion of an amino acid chain.

The term "proteinaceous compound" as used herein relates to any peptide, oligopeptide, polypeptide, monomeric protein, multimeric protein or multisubunit protein complex.

The expression "ligand-binding proteinaceous compound" as used herein relates to any proteinaceous compound having the ability to specifically interact with a certain ligand, resulting in a biding of the ligand by the proteinaceous compound. In a preferred embodiment of the present invention, the proteinaceous compound is a lectin.

The term "matrix" as used herein relates to any solid carrier material having the ability to bind and present a ligand which is inert to the procedural steps of the above method, especially the proteolytically degradation of the proteinaceous compound in the above method. The term "matrix" does not have any specific limitations, and relates, for example, to an insoluble polymer material, which can be an organic polymer, such as polyamide or a vinyl polymer (e.g. poly(meth)acrylate, polystyrene and polyvinyl alcohol, or derivatives thereof), a natural polymer such as cellulose, dextrane, agarose, chitin and polyamino acids, or an inorganic polymer, such as glass or metallohydroxide. The matrix can be in the form of a microcarrier, particles, membranes, strips, paper, film, pearls or plates, such as microtiter plates or microarrays. The term "microarray" as used herein means any arrangement of ligands in addressable locations on a matrix resulting in a so-called "biochip". An example of the immobilization matrix is epoxy-activated Sepharose 6B.

The term "proteolytical" as used herein relates to any form of degradation of a proteinaceous compound. Means of proteolytical degradation according to the present invention include chemical and enzymatical degradation. For example the proteolytical degradation is carried out enzymatically. The enzymatically degradation can be carried out using for example an enzyme being selected from the group, consisting of aminopeptidases, carboxypeptidases, cathepsins, endopeptidases, exopeptidases, metalloproteases, serine proteases, chymotrypsin, papain, pepsin, Pronase, proteinase K, trypsin, ArgC- protease, Lys-C protease, Glu-C protease, dipeptidyl-aminopeptidase, and any mixture combination of these proteases. In a preferred embodiment of the present invention the enzyme is trypsin and/or Pronase.

The expression "ligand recognition domain (LRD)-containing fragment" as used herein relates to any fragment of a proteinaceous compound comprising at least one of the proteinaceous compound's LRD. In a preferred embodiment of the present invention, the LRD is a carbohydrate recognition domain (CRD) and the ligand comprises a carbohydrate moiety.

The expression "altered ligand recognition" as used herein relates to any significantly strengthened, weakened or completely abolished binding of a proteinaceous compound to its ligand which might be caused by for example an altered amino acid sequence of the proteinaceous compound or conformational changes which might be due to for example disease-related changes in the processing of the proteinaceous compound.

The term "sample" as used herein relates to any composition that contains or is suspected of containing a proteinaceous compound according to the present invention. Examples for such a sample are whole blood, serum, plasma, urine or liquor of an individual or eukaryotic or prokaryotic cell extracts or plant tissues. Further, the sample may comprise a solution derived from naturally occurring systems, e.g. a solution containing isolated blood compounds or processed blood products. In one embodiment of the present invention, the sample may comprise cells expressing the proteinaceous compound, e.g. a cell culture system. Methods for obtaining the above samples are known in the prior art.

In a preferred embodiment of the present invention, step (a) of the above methods is further carried out under conditions suitable for maintaining the native form of the proteinaceous compound.

In another preferred embodiment of the present invention, the above methods further comprise at least one washing step. In a more preferred embodiment, the above methods comprise the step of:
(i) washing the immobilized ligand after contacting it with the proteinaceous compound and analyzing the washing fraction by mass spectrometry (MS); and/ or
(ii) washing the immobilized ligand after proteolytical degradation of the proteinaceous compound to obtain the non-LRD-containing fragments of the proteinaceous compound and analyzing these by mass spectrometry.

In one embodiment of the present invention, releasing the ligand-bound LRD-containing fragment(s) of the proteinaceous compound from the ligand being fixed to the matrix comprises competitive elution. The term "competitive elution" as used herein relates to any method of releasing a ligand-bound compound from the ligand by using a compound that can competitively bind to the ligand-bound compound, using the same binding site thus displacing the immobilized ligand.

In another embodiment of the present invention, mass spectrometry comprises electrospray ionization (ESI) and/or matrix assisted laser desorption/ionization (MALDI). These so called "soft" ionization/desorption techniques are suitable to directly analyze intact peptide/protein-ligand interactions and to yield stable, intact and highly accurate molecular mass data of polypeptides, carbohydrates and nucleic acids. In a preferred embodiment of the present invention, mass spectrometry comprises time of flight mass spectrometry (TOF-MS) and/or Fourier transform ion cyclotron resonance mass spectrometry (FTICR-MS).

An example of the present invention is the so-called CREDEX-MS (carbohydrate recognition domain excision mass spectrometry). In this embodiment, the ligand-binding proteinaceous compound according to the present invention is a carbohydrate binding protein. In a preferred embodiment of the present invention, the carbohydrate binding protein is selected from the group consisting of lectins, anti-carbohydrate antibodies, carbohydrate binding enzymes and carbohydrate binding transport proteins. In a more preferred embodiment of the present invention, the carbohydrate binding protein is a lectin. Accordingly, the ligand in CREDEX-MS comprises a carbohydrate moiety. In a more preferred embodiment, the carbohydrate moiety is lactose.

In another embodiment of the present invention, the proteinaceous compound is bound to a cell. The expression "proteinaceous compound presented by a cell" as used herein relates to any proteinaceous compound that is directly or indirectly bound on the surface of a cell. The cell may be for example selected from the group consisting of mammalian cells, plant cells, insect cells, bacterial cells, protozoae, viruses, fungi, or neuronal cells. In a preferred embodiment of the present invention, the cell is a tumor cell.

In one embodiment, the present invention relates to a biochip comprising at least one immobilized ligand for the identification and/or determination of at least one ligand recognition domain (LRD) of a ligand-binding proteinaceous compound, wherein the ligand comprises a carbohydrate moiety or a nucleic acid moiety according to the present invention. In a preferred embodiment of the present invention, the biochip comprises only one type of ligand. In another preferred embodiment of the present invention, the biochip comprises at least two different types of ligand.

In another embodiment, the present invention relates to the use of a biochip comprising at least one immobilized ligand in the method for the identification and/or determination of at least one ligand recognition domain (LRD) of a ligand-binding proteinaceous compound according to the present invention.

In a further embodiment, the present invention relates to the use of a biochip comprising at least one immobilized ligand in the diagnostic assay for detecting a disorder associated with an increase or decrease of a ligand-binding proteinaceous compound and/or with an altered ligand recognition of a ligand-binding proteinaceous compound according to the present invention.

The present invention also relates to a kit for carrying out the above method, comprising a proteolytically stable ligand immobilized on a matrix, a suitable elution buffer, and a proteolytic agent. In a preferred embodiment, the kit further comprises at least one suitable washing buffer.

Further, the present invention relates to a kit comprising means for carrying out at least one of the methods according to the present invention. Means for carrying out the method for the identification and/or determination of at least one ligand recognition domain (LRD) of a ligand-binding proteinaceous compound include, but are not limited to, suitable buffers, ligands, reagents, consumables, matrices and biochips.

Moreover, the present invention relates to a diagnostic kit comprising means for carrying out at least one of the diagnostic assays according to the present invention. Means for carrying out the diagnostic assay for detecting a disorder associated with an increase or decrease of a ligand-binding proteinaceous compound and/or with an altered ligand recognition of a ligand-binding proteinaceous compound include, but are not limited to, suitable buffers, reagents, consumables, matrices and biochips.

In one embodiment, the present invention relates to a peptide comprising the amino acid sequence of a ligand recognition domain (LRD) of a proteinaceous compound obtainable by any of the preceding methods according to the present invention. In a preferred embodiment of the present invention, the peptide has a length of 8 to 20 amino acids, more preferred of 5 to 12 amino acids, and most preferred of 5 to 7 amino acids.

In another embodiment, the present invention relates to a method for the preparation of peptides comprising the amino acid sequence of a ligand recognition domain (LRD) of a proteinaceous compound identified by any of the preceding methods according to the present invention comprising solid phase peptide synthesis. In one embodiment of the present invention, the peptide is a peptide as defined above.

Additionally, the present invention relates to a method for the identification and/or determination of at least one ligand-containing molecule in a sample comprising the steps of:
(a) contacting said sample with at least one ligand recognition domain (LRD)-containing peptide obtainable by any of the above methods immobilized on a matrix under conditions suitable for binding said ligand-containing molecule to said peptide;
(b) releasing the LRD-bound ligand-containing molecules obtained in step (a) from said peptide; and
(c) analyzing the ligand-containing molecules obtained in step (b).

In another aspect, the present invention relates to a diagnostic assay for detecting a disorder associated with an increase or decrease of a specific ligand comprising the steps of:
(a) contacting a sample containing at least one ligand-containing molecule with at least one ligand recognition domain (LRD)-containing peptide according to the present invention immobilized on a matrix under conditions suitable for binding said ligand-containing molecule to said peptide;
(b) releasing the LRD-bound ligand-containing molecules obtained in step (a) from said peptide; and
(c) analyzing the ligand-containing molecule obtained in step (b).

The terms "ligand", "ligand recognition domain", "proteinaceous compound", "sample", "ligand recognition domain (LRD)-containing peptide, and "matrix" as used herein are defined as defined above.

The term "ligand-containing molecule" as used herein relates to any compound containing at least one moiety that can bind to a ligand recognition domain (LRD)-containing peptide according to the present invention.

The increase or decrease of a specific ligand may be due to any alteration of physiological functions in the individual from which the sample is taken. For example the increase or decrease of a specific ligand may be due to a reduction or activation of glycosylation mechanisms or an altered expression of the specific ligand. The expression "altered expression of the specific ligand" as used herein relates to any change in the expression of a specific ligand that leads to significantly strengthened, weakened or completely abolished binding of the ligand to a ligand recognition domain (LRD)-containing peptide. For example if changes in a glycosylation pattern of a cell occur, a ligand can turn into a different ligand being specific for a diseased cell which is a viable ligand for a different LRD.

The analyzing of the ligand-containing molecule can be carried out by all suitable methods known in the art. For example, the ligand-containing molecules can be evaluated qualitatively and/or quantitatively by methods well known in the art. Examples for the evaluation of the ligand-containing molecules include, but are not limited to, mass spectrometry, sequencing, SPR, NMR, XRD, ELISA, HPLC, gel electrophoresis, Edman sequencing, BCA assay, and various ligand-specific chemical tests.

In one embodiment of the present invention, at least steps (a) and (b) of the above methods are carried out under conditions suitable for maintaining the native form of the ligand.

In a preferred embodiment of the present invention, the above methods further comprise at least one washing step. In a more preferred embodiment, the above methods comprise the step of washing the immobilized ligand recognition domain (LRD)-containing peptide after contacting it with the sample and analyzing the washing fraction by mass spectrometry (MS).

In another preferred embodiment of the present invention, releasing the LRD-bound ligand-containing molecules from the peptide comprises competitive elution. The term "competitive elution" as used herein relates to any method of releasing a ligand-bound compound from the ligand by using a compound that can competitively bind to the ligand-bound compound, the compound being the same as the ligand or different from it.

The ligand-containing molecule may be bound to a cell. The expression "molecule bound to a cell" as used herein relates to any compound that is directly or indirectly bound on the surface of a cell. The cell may be selected from the group consisting of mammalian cells, plant cells, insect cells, bacterial cells, protozoae, viruses, fungi, or neuronal cells. In a preferred embodiment of the present invention, the cell is a tumor cell.

Further, the present invention relates to the use of ligand recognition domain (LRD)-containing peptides according to the present invention in the manufacture of a biochip comprising at least one immobilized ligand recognition domain (LRD)-containing peptide obtainable by any of the above methods for the identification and/or determination of ligand-containing molecules in a sample.

Moreover, the present invention relates to a biochip comprising at least one immobilized ligand recognition domain (LRD)-containing peptide obtainable by any of the above methods for the identification and/or determination of ligand-containing molecules in a sample. In a preferred embodiment of the present invention, the biochip comprises only one type of ligand. In another preferred embodiment of the present invention, the biochip comprises at least two different types of ligand.

In one embodiment, the present invention relates to the use of a biochip comprising at least one immobilized ligand recognition domain (LRD)-containing peptide according to the present invention in the method for the identification and/or determination of at least one ligand-containing molecule in a sample according to the present invention.

In another embodiment, the present invention relates to the use of a biochip comprising at least one immobilized ligand recognition domain (LRD)-containing peptide according to the present invention in the diagnostic assay for detecting a disorder associated with an increase or decrease of a specific ligand and/or with an altered expression of a specific ligand according to the present invention.

Additionally, the present invention relates to a kit comprising means for carrying out at least one of the methods according to the present invention. Means for carrying out the method for the identification and/or determination of at least one ligand-containing molecule in a sample include, but are not limited to, suitable buffers, reagents, consumables, matrices and biochips.

In one embodiment, the present invention relates to a diagnostic kit comprising means for carrying out at least one of the diagnostic assays according to the present invention. Means for carrying out the diagnostic assay for detecting a disorder associated with an increase or decrease of a specific ligand and/or with an altered expression of a specific ligand include, but are not limited to, suitable buffers, reagents, consumables, matrices and biochips.

The figures show:

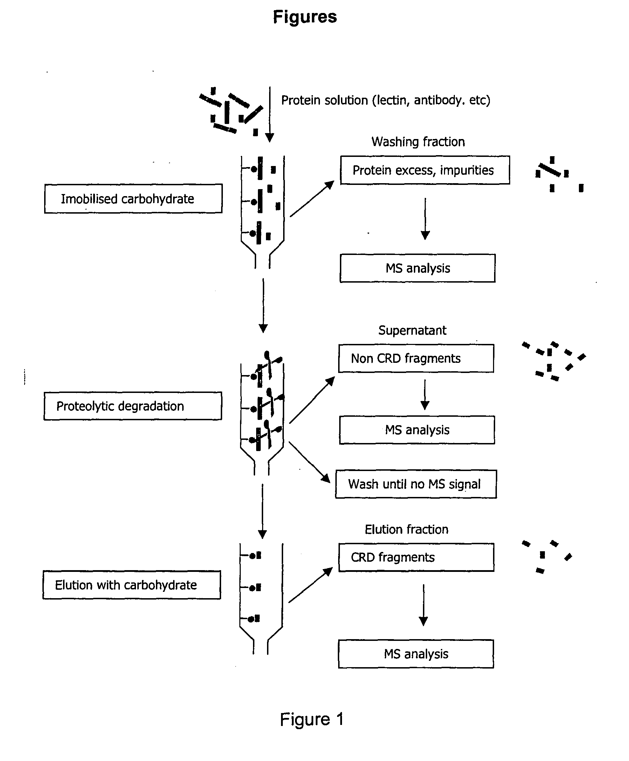

Figure 1: Principle of CREDEX-MS: Analytical concept and scheme for proteolytic-excision mass spectrometric identification of LRD sequences. CREDEX-MS consists of the proteolytic excision and mass spectrometric identification of LRD sequences. The ligand-binding is bound on a column with immobilised ligand and digested with proteases. The protein fragments not binding to the ligand are washed from the column and analyzed by FTICR-MS. The remaining bound fragments are eluted with the same or a different ligand (competitive elution) and analyzed by mass spectrometry. The native conformation of the protein is kept by running the experiment in physiological conditions.

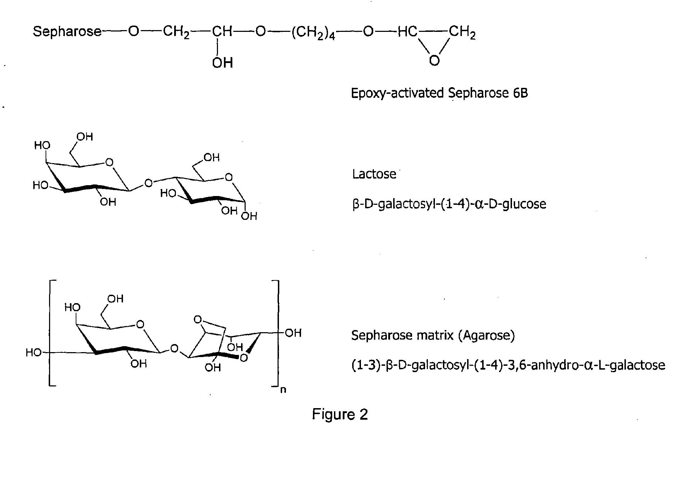

Figure 2: Epoxy activated Sepharose 6B is the immobilization matrix used for carbohydrate immobilisation. The Sepharose 6B contains 6% (w/v) agarose which is a polysaccharide containing β-D-galactose. All lectins and antibodies capable of recognizing and binding to β-D-galactose and oligo and polysaccharides which contain β-D-galactose have to be tested for Sepharose affinity in order to eliminate a false-positive response.

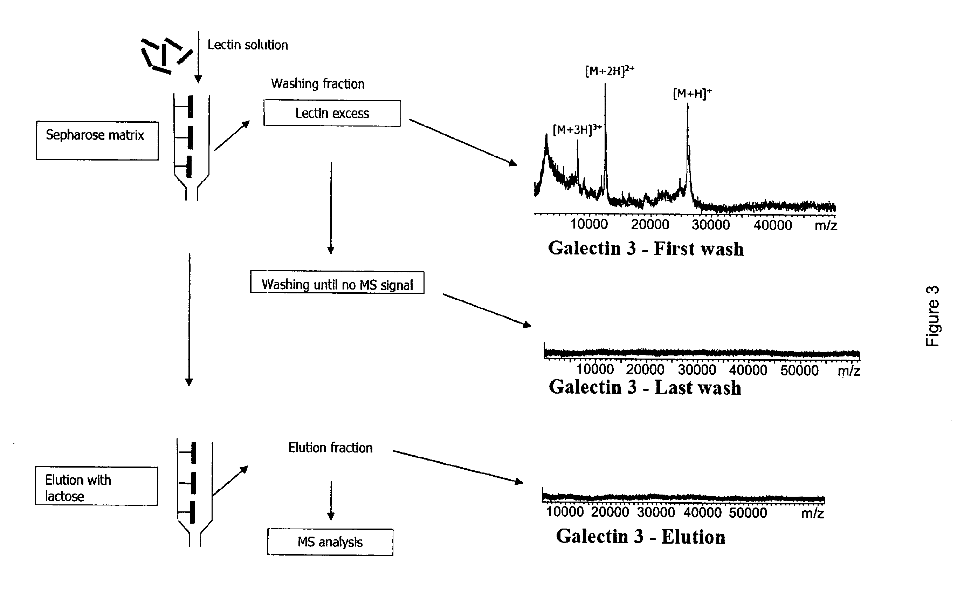

Figure 3: Galectin-3 interaction study with Sepharose matrix by affinity MS as negative control. Since the galectins specifically recognize β-D-galactose, a galectins-Sepharose affinity test is necessary in order to eliminate a false-positive response.

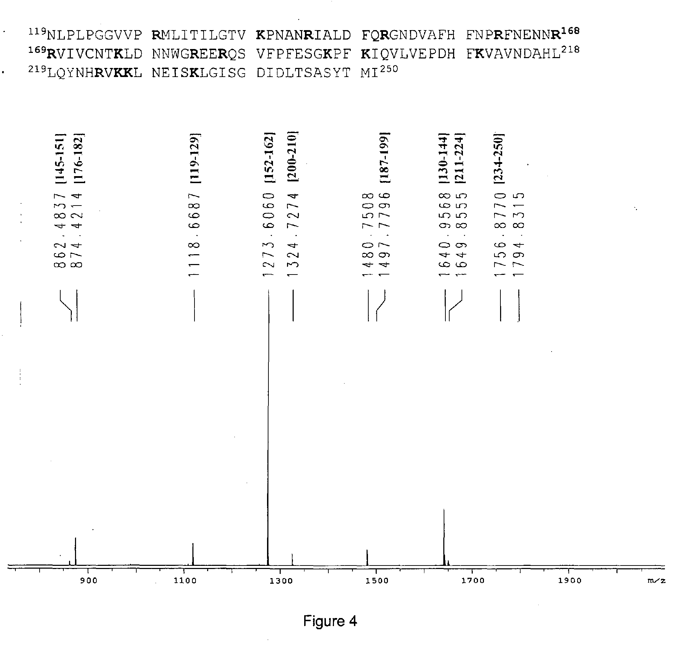

Figure 4: FTICR-MS of tryptic peptide fragments of galectin-3. In solution digestion was performed on 100 µg galectin-3 using trypsin (enzyme : substrate ratio 1:50) for three hours at 37°C in 100 µL PBS 50mM pH 7.5.

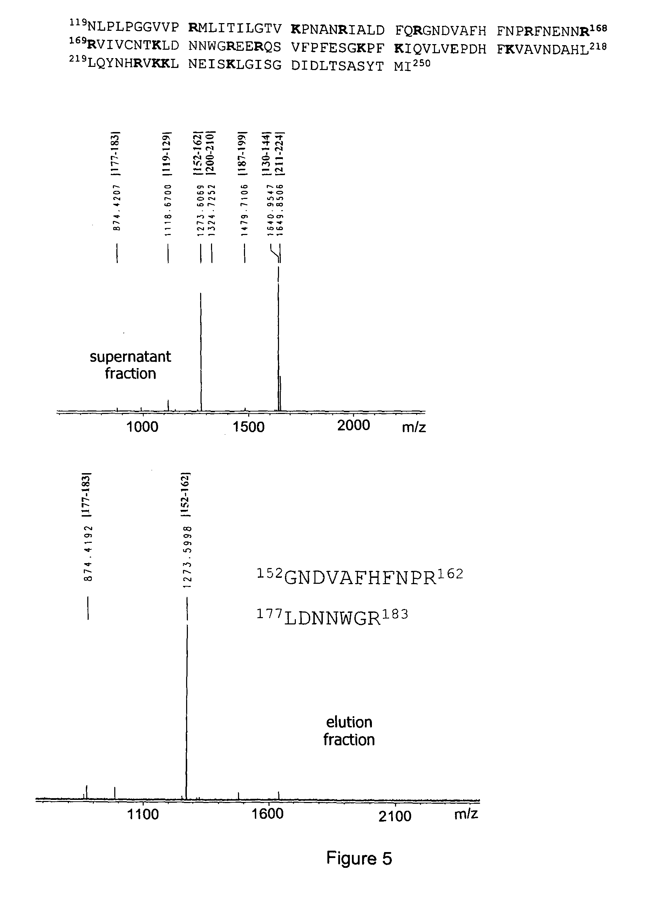

Figure 5: CREDEX-MS of galectin-3 interaction with lactose provides two specific LRD peptides (152-162) and (177-183). The upper spectrum shows the fragments identified in the supernatant fraction and the lower spectrum shows the fragments identified in the elution fraction (the LRD peptides (152-162) and (177-183)). FTICR-MS was obtained with a Bruker Apex II 7T FT-ICR mass spectrometer (Bruker Daltonik, Bremen, Germany).

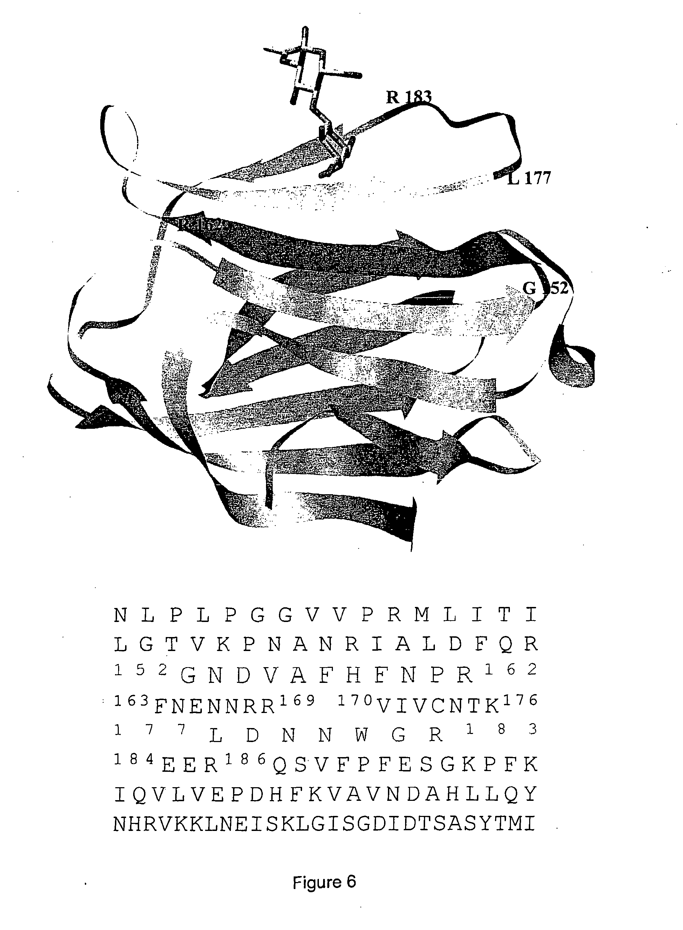

Figure 6: Topology of LRD peptides identified by CREDEX-MS (152-162) and (177-183) in the crystal structure of galectin-3. The molecular graphics image presented was produced using the UCSF Chimera package from the Resource for Biocomputing, Visualization, and Informatics at the University of California, San Francisco (supported by NIH P41 RR-01 081).

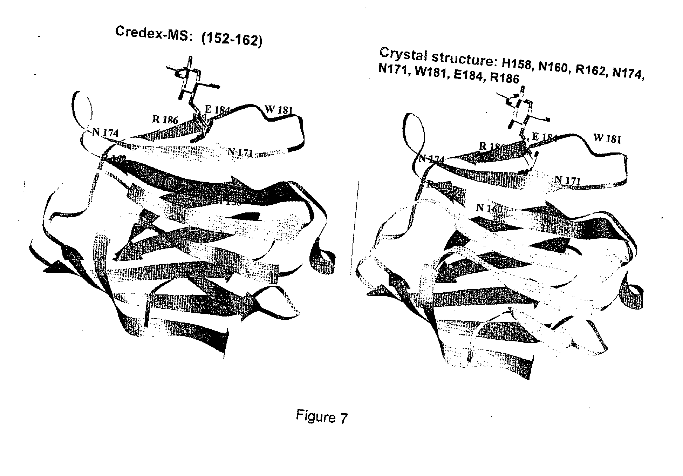

Figure 7: Comparison of CREDEX-MS and crystallographic data of galectin-3 LRD. On the left side is the crystal structure of galectin-3 with the peptides identified by CREDEX-MS (152-162) and (177-183). On the right side is the crystal structure of galectin-3 with the binding site of the carbohydrate (152-186). The amino acids that are in direct contact with the carbohydrate are marked in bold (H158, N160, R162, N174, N171, W181, E184, R186). The molecular graphics images presented were produced using the UCSF Chimera package from the Resource for Biocomputing, Visualization, and Informatics at the University of California, San Francisco (supported by NIH P41 RR-01081).

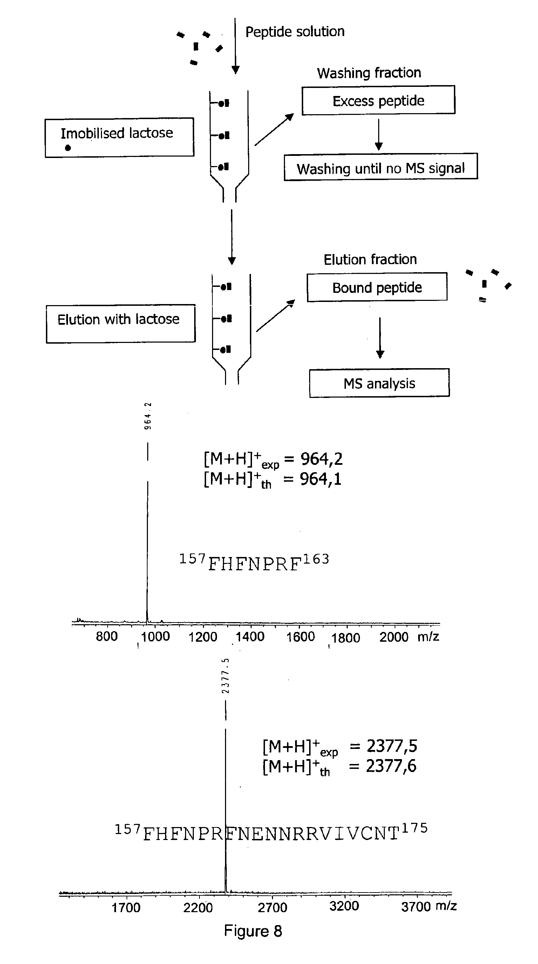

Figure 8 a, b, c: Characterization of synthetic LRD peptides by affinity MS. Two galectin-3 peptides (157-163) and (157-175) were synthesized by Fmoc-SPPS, purified by RP-HPLC and characterized by MALDI-TOF-MS. The synthetic peptides solutions were added to the lactosyl-Sepharose column and after 12 hours shaking at 37°C the supernatant was analysed by MALDI-MS. The column was washed with the binding solvent until the washing fractions did not present any signals when analysed by MALDI-MS. Lactose elution was performed with 400 µL 0.3 M lactose in PBS pH 7.5 shaking at 37°C for 15 min, and elution fractions analyzed by MALDI-MS.

One goal of the present invention is to provide a method for the determination of relevant ligand recognition domain (LRD) peptide sequences from ligand-binding proteins with high sensitivity and by application of the molecular specificity of mass spectrometry. The combination of specific proteolytic excision of affinity-bound proteins and mass spectrometric analysis of the eluted peptides has been shown to be a highly efficient tool for elucidating specific LRD structures. The method is generally applicable to a wide range of ligand-binding proteins and is feasible to a wide range of non-proteinaceous and non-peptidic ligands that can be immobilised on a solid surface or related substrate. Furthermore, the method is feasible with a range of soft ionisation/desorption methods of mass spectrometry, such as electrospray ionization (ESI) and matrix assisted laser desorption/ionisation (MALDI), and a wide range of mass spectrometer types, such as quadrupole, ion-trap and time-of-flight analysers, although the use of high resolution FTICR-MS is of particular advantage for unequivocal peptide identification. The method is also of clinical relevance in the diagnosis of disorders that are associated with qualitative and/or quantitative alterations in the expression of a ligand-binding protein.

An innovative aspect of the invention is that it provides *inter alia* the direct identification, and access to molecular chemical structures, in the form of LRD-containing peptides, that are relevant for specific ligand affinity of proteins. Peptide identification is fast, easy, requires only minimal amounts of starting material and can be achieved directly out of complex starting materials such as cells. For a given ligand, all proteins and their ligand recognition domains that are involved in interactions with that ligand can be identified. The corresponding peptides represent useful model structures which are readily amenable to chemical synthesis, thus facilitating the study, mechanistic understanding and biomedical applications of ligand-binding biomolecules, as well as the use of such peptides as lead compounds in the development of new diagnostic and therapeutic drugs. Making use of such peptides, the present invention also provides a method for the identification of ligand-containing molecules in a sample. This method can be used in the diagnosis of disorders that are associated with qualitative and/or quantitative alterations in the expression of a specific ligand.

The present invention will now be further illustrated in the following examples without being limited thereto.

### Examples

### Example 1: The ligand recognition domains of lactose-binding Galectin-3

### Interaction of Galectin-3 with Sepharose matrix as negative control

Epoxy-activated Sepharose 6B is the immobilization matrix used for the lactose immobilisation. Sepharose 6B contains 6% (w/v) agarose which is a polysaccharide containing β-D-galactose. All proteins capable of recognizing and binding to β-D-galactose and/or oligo- and polysaccharides which contain β-D-galactose have to be tested for Sepharose affinity in order to eliminate a false-positive response.

The matrix preparation for the control test was made as follows: Epoxy-activated Sepharose 6B was subjected to swelling in MilliQ water for 1 hour, with 2 changes of solution. The resulting gel was washed for 1 hour on a sintered glass filter. The gel was mixed 1:1 with 0.2 M K2HP04 pH 13 and shaken at 37°C for 24 hours. The gel was then washed using 100 mL of the same buffer. A blocking step followed, with 1 M ethanolamine pH 8, in a shaker at 40°C overnight. The gel was then washed with 100 mL 0.2 M K₂HPO₄ pH 13 followed by 4 cycles alternative washing with volumes of 10 mL buffers of different pH (0.1 M acetate buffer containing 0.5 M NaCl pH 4 and 0.1 M Tris-HCl buffer containing 0.5 M NaCl pH 8). The gel was then equilibrated with 50 mL PBS 50 mM pH 7.5.

For the galectin-3 negative control a solution of 100 µg galectin-3 in 100 µL PBS 50 mM pH 7.5 was mixed with 200 µL pure Sepharose gel (prepared as specified above) and shaken gently at 37°C for 24 h. The supernatant was collected and analysed by MALDI-TOF-MS in order to verify the extent of binding. The free (unbound) lectin was washed out with binding buffer (30-40 mL) and the washing fractions were analysed by MALDI-TOF-MS. The next step was an elution with lactose, 400 µL 0.3 M lactose in PBS pH 7.5 shaking at 37°C for 15 min. This step was repeated two times. This was followed by another washing step with 1.2 mL 0.3 M lactose in PBS pH 7.5. The elution fractions were analysed by MALDI-TOF-MS. The mass spectrum of the first washing fraction with the signals for the single, double and triple charged ions can be seen. The MALDI-TOF spectra of the final washing fraction and elution fraction presented no signal. This proves that Sepharose is a suitable matrix, since no binding of Galectin-3 was observed.

### Immobilisation of lactose to epoxy-activated Sepharose

Epoxy-activated Sepharose 6B was subjected to swelling in MilliQ water for 1 hour, with 2 changes of solution. The resulting gel was washed for 1 hour on a sintered glass filter. For the lactose immobilisation the gel was mixed with a solution of 50 mg lactose for each mL of gel in 0.2 M K₂HPO₄ pH 13 and shaken at 37°C for 24 hours. The excess ligand was washed using the coupling buffer (100 mL). The remaining active epoxy groups were blocked with 1 M ethanolamine pH 8, in a shaker at 40°C overnight. The gel was then washed with 100 mL coupling buffer followed by 4 cycles alternative washing with volumes of 10 mL buffers of different pH (0.1 M acetate buffer containing 0.5 M NaCl pH 4 and 0.1 M Tris-HCl buffer containing 0.5 M NaCl pH 8) in order to remove the electrostatically bound lactose. The gel was then equilibrated with 50 mL binding buffer (PBS 50 mM pH 7.5 ).

### Identification of the lactose recognition domains in Galectin-3

A solution of 100 µg galectin-3 /100 µL PBS 50 mM pH 7.5 was mixed with 200 µL lactosyl-Sepharose gel and shaken gently at 37°C for 24 h. The supernatant was collected and analyzed by MALDI-TOF-MS in order to verify the extent of binding. The free (unbound) Galectin-3 was washed out with binding buffer (30 to 40 mL) and the washing fractions were analysed by MALDI-TOF-MS. The digestion of bound galectin was performed using trypsin, in the binding buffer, with an enzyme to substrate ratio of 1:100, at 37°C, for 3 h. After digestion, the supernatant was measured by MALDI-FTICR-MS. The free peptides (not bound to lactose) were washed out with 30 to 40 mL PBS pH 7.5. The washing fractions were analysed by MALDI-FTICR-MS. The peptides bound to lactose were eluted by shaking three times with 400 µL 0.3 M lactose in PBS pH 7.5 at 37°C for 15 min. This was followed by another washing step with 1.2 mL 0.3 M lactose in PBS pH 7.5. The eluate was analyzed by MALDI-FTICR-MS.

### Mass spectrometry

All FTICR-MS spectra were obtained with a Bruker Apex II 7T FT-ICR mass spectrometer (Bruker Daltonik, Bremen, Germany) equipped with an external Scout 100 fully-automated X-Y target stage MALDI source with pulsed collision gas. The pulsed nitrogen laser was operated at 337 nm. Ions generated by laser shots were accumulated in the hexapole for 0.5-1 sec at 15 V and extracted at -7 V into the analyzer cell. A 100 mg/ml solution of 2,5-dihydroxybenzoic acid (DHB, Aldrich, Germany) in acetonitrile: 0.1% TFA in water (2:1) was used as the matrix. 0.5 µL of sample solution were mixed on the stainless-steel MALDI sample target and allowed to dry.

### Synthesis of ligand recognition domain-containing Galectin-3 peptides

Peptides Gal-3₁₅₇₋₁₆₃ (¹⁵⁷FHFNPRF¹⁶³) and Gal-3₁₅₇₋₁₇₅ (¹⁵⁷FHFNPRFNENNRRVIVCNT¹⁷⁵) were synthesized by solid-phase peptide synthesis (SPPS) on a NovaSyn TGR resin, containing a polystyrene-polyethyleneglycol resin and a Rink-amide-linker cleavable under acidic conditions, according to commercially available material and published literature procedures. 9-Fluorenylmethoxycarbonyl/t-butyl (Fmoc/tBu) chemistry was used throughout the synthesis using a semi-automated Economy Peptide Synthesizer EPS-221 (ABIMED, Germany). The following N(-Fmoc) and side-chain protected amino acid derivatives were used: Fmoc-Ala-OH, Fmoc-Cys(Trt)-OH, Fmoc-Asp(OtBu)-OH, Fmoc-Glu(OtBu)-OH, Fmoc-Phe-OH, Fmoc-Gly-OH, Fmoc-His(Trt)-OH, Fmoc-Ile-OH, Fmoc-Lys(Boc)-OH, Fmoc-Asn(Trt)-OH, Fmoc-Pro-OH, Fmoc-Gln(Trt)-OH, Fmoc-Arg(Pbf)-OH, Fmoc-Ser(tBu)-OH, Fmoc-Thr(tBu)-OH, Fmoc-Val-OH, Fmoc-Trp(Boc)-OH. The synthesis was performed according to the following general protocol: (i) DMF washing (3x1 min), (ii) Fmoc deprotection with 2% DBU, 2% piperidine in DMF (15 min), (iii) DMF washing (6x1 min), (iv) coupling of 5 equivalents of Fmoc amino acid : PyBOP : NMM in DMF (40 to 60 min), (v) DMF washing (3x1 min).
After completion of the syntheses, the peptides were cleaved from the resin using a TFA, triethylsilane and deionized water mixture (95:2.5:2.5, V/V/V) for 3 h at room temperature. The crude product was precipitated with cold tert-butylmethylether, washed three times with diethyl ether and solubilized in 10% acetic acid (aqueous solution) prior to freeze-drying. Purification of the peptides was performed by semipreparative HPLC. Subsequent characterisation by HPLC and MALDI-TOF mass spectrometric analysis ensured molecular homogeneity of the peptide. The synthetic peptides were used for affinity-MS characterisation.

### Characterization of synthetic ligand recognition domain-containing Galectin-3 peptides by MS

Two galectin-3 peptides (157-163) and (157-175) were synthesized by Fmoc-SPPS, purified by RP-HPLC and characterized by MALDI-TOF-MS. The synthetic peptides solutions were added over the lactosyl-Sepharose columns and after 12 hours shaking at 37 °C the supernatant was analysed by MALDI-TOF. The columns were washed with the binding solvent until the washing fractions did not present any signals when analysed by MALDI-TOF. After this, the lactose elution was performed with 400 µL 0.3 M lactose in PBS pH 7.5 shaking at 37°C for 15 min and repeated two times. The elution fractions for both peptides, (157-163) and (157-175), presented signals (Figure 8 a and b). This experiment shows that the peptides chosen for synthesis indeed present affinity for lactose thus supporting the CREDEX-MS results.

## Claims

1. A method for the identification and/or determination of at least one ligand recognition domain (LRD) of a ligand-binding proteinaceous compound, comprising the steps of:
(a) contacting said proteinaceous compound with a proteolytically stable ligand immobilized on a matrix under conditions suitable for binding said proteinaceous compound to said ligand;
(b) proteolytically degrading the proteinaceous compound bound to the immobilized ligand of step (a);
(c) releasing the ligand-bound LRD-containing fragment(s) of the proteinaceous compound obtained in step (b) from the ligand; and
(d) analyzing the fragment(s) obtained in step (c) using mass spectrometry (MS).

2. The method according to claim 1, wherein the ligand comprises a carbohydrate moiety of a nucleic acid moiety.

3. The method according to claim 1 or 2, wherein the proteolytical degradation of step (b) is carried out enzymatically.

4. The method according to claim 3, wherein the enzyme is trypsin and/or Pronase.

5. The method according to any of claims 1 to 4, wherein the releasing of the ligand-bound LRD-containing fragment(s) from the ligand in step (c) is carried out by competitive elution.

6. A kit for carrying out the method according to any of claims 1 to 5, comprising a proteolytically stable ligand immobilized on a matrix, a suitable elution buffer, and a proteolytic agent.

7. A method for the identification and/or determination of at least one ligand-containing molecule in a sample comprising the steps of:
(a) contacting said sample with at least one ligand recognition domain (LRD)-containing peptide obtainable by the method of any of claims 1 to 5 immobilized on a matrix under conditions suitable for binding said ligand-containing molecule to said peptide;
(b) releasing the LRD-bound ligand-containing molecules obtained in step (a) from said peptide; and
(c) analyzing the ligand-containing molecule obtained in step (b).

8. The method according to claim 7, wherein the releasing of the LRD-bound ligand-containing molecule from said peptide in step (b) is carried out by competitive elution.

9. The method according to any of claims 7 or 8, wherein the ligand-containing molecule is presented by a cell, selected from the group consisting of mammalian cells, plant cells, insect cells and bacterial cells.

10. The method of claim 9, wherein the cell is a tumor cell.

11. The method according to any of claims 1 to 5 and 7 to 10, wherein the ligand comprises a carbohydrate moiety or a nucleic acid moiety.

12. A biochip comprising at least one immobilized ligand recognition domain (LRD)-containing peptide obtainable by the method of any of claims 1 to 5 for the identification and/or determination of ligand-containing molecules in a sample.

13. A peptide comprising the amino acid sequence of a ligand recognition domain (LRD) of a proteinaceous compound obtainable by the method of any of claims 1 to 5.


SEQUENCE LISTING

<110>  Steinbeis-Transferzentrum

<120>  Identification of ligand recognition domains

<130>  S 9247EU

<160>  8

<170>  PatentIn version 3.5

<210>  1
<211>  11
<212>  PRT
<213>  Artificial Sequence

<220>
<223>  peptide

<400>  1

Gly Asn Asp Val Ala Phe His Phe Asn Pro Arg
1               5                   10


<210>  2
<211>  7
<212>  PRT
<213>  Artificial Sequence

<220>
<223>  peptide

<400>  2

Leu Asp Asn Asn Trp Gly Arg
1               5


<210>  3
<211>  7
<212>  PRT
<213>  Artificial Sequence

<220>
<223>  peptide

<400>  3

Phe His Phe Asn Pro Arg Phe
1               5

<210>  4
<211>  19
<212>  PRT
<213>  Artificial Sequence

<220>
<223>  peptide

<400>  4

Phe His Phe Asn Pro Arg Phe Asn Glu Asn Asn Arg Arg Val Ile Val
1               5                   10                  15

Cys Asn Thr

<210>  5
<211>  50
<212>  PRT
<213>  Artificial Sequence

<220>
<223>  peptide

<400>  5

Asn Leu Pro Leu Pro Gly Gly Val Val Pro Arg Met Leu Ile Thr Ile
1               5                   10                  15

Leu Gly Thr Val Lys Pro Asn Ala Asn Arg Ile Ala Leu Asp Phe Gln
            20                  25                  30

Arg Gly Asn Asp Val Ala Phe His Phe Asn Pro Arg Phe Asn Glu Asn
        35                  40                  45

Asn Arg
    50

<210>  6
<211>  50
<212>  PRT
<213>  Artificial Sequence

<220>

<223>  peptide

<400>  6

Arg Val Ile Val Cys Asn Thr Lys Leu Asp Asn Asn Trp Gly Arg Glu
1               5                   10                  15

Glu Arg Gln Ser Val Phe Pro Phe Glu Ser Gly Lys Pro Phe Lys Ile
            20                  25                  30

Gln Val Leu Val Glu Pro Asp His Phe Lys Val Ala Val Asn Asp Ala
        35                  40                  45

His Leu
    50

<210>  7
<211>  32
<212>  PRT
<213>  Artificial Sequence

<220>
<223>  peptide

<400>  7

Leu Gln Tyr Asn His Arg Val Lys Lys Leu Asn Glu Ile Ser Lys Leu
1               5                   10                  15

Gly Ile Ser Gly Asp Ile Asp Leu Thr Ser Ala Ser Tyr Thr Met Ile
            20                  25                  30

<210>  8
<211>  131
<212>  PRT
<213>  Artificial Sequence

<220>
<223>  peptide

<400>  8

Asn Leu Pro Leu Pro Gly Gly Val Val Pro Arg Met Leu Ile Thr Ile
1               5                   10                  15

Leu Gly Thr Val Lys Pro Asn Ala Asn Arg Ile Ala Leu Asp Phe Gln
            20                  25                  30

Arg Gly Asn Asp Val Ala Phe His Phe Asn Pro Arg Phe Asn Glu Asn
        35                  40                  45

Asn Arg Arg Val Ile Val Cys Asn Thr Lys Leu Asp Asn Asn Trp Gly
    50                  55                  60

Arg Glu Glu Arg Gln Ser Val Phe Pro Phe Glu Ser Gly Lys Pro Phe
65                  70                  75                  80

Lys Ile Gln Val Leu Val Glu Pro Asp His Phe Lys Val Ala Val Asn
                85                  90                  95

Asp Ala His Leu Leu Gln Tyr Asn His Arg Val Lys Lys Leu Asn Glu
            100                 105                 110

Ile Ser Lys Leu Gly Ile Ser Gly Asp Ile Asp Thr Ser Ala Ser Tyr
        115                 120                 125

Thr Met Ile
    130